Europäisches Patentamt

European Patent Office

Office européen des brevets

(19)

(11) Publication number:

**0 286 351**
**A2**

# (12) EUROPEAN PATENT APPLICATION

(21) Application number: 88303008.2

(51) Int. Cl.⁴: **C05F 11/08 , C12M 1/04**

(22) Date of filing: 05.04.88

(30) Priority: 07.04.87 GB 8708273

(43) Date of publication of application:
12.10.88 Bulletin 88/41

(84) Designated Contracting States:
AT BE CH DE ES FR GB GR IT LI LU NL SE

(71) Applicant: **AGRICULTURAL GENETICS
COMPANY LIMITED
Unit 154/155 Cambridge Science Park Milton
Road
Cambridge CB4 4BH(GB)**

(72) Inventor: **Day, John Michael
7 East Common Harpenden
Hertfordshire AL5 1BJ(GB)**
Inventor: **Williams, Paul Michael
Flat 5 Rothamstead Manor
Harpenden Hertfordshire AL5 2JQ(GB)**

(74) Representative: **Lewin, John Harvey et al
Elkington and Fife High Holborn House 52/54
High Holborn
London WC1V 6SH(GB)**

(54) Granular inoculant composition for plants.

(57) This invention provides a method of preparing a granular inoculant composition for plants, which comprises mixing at least one species of microorganism capable of having a beneficial effect on the plants with a peat or peat/clay carrier medium; mixing the product with calcium sulphate and moistening to cause aggregation; and drying to produce free-flowing granules.

EP 0 286 351 A2

## Granular inoculant composition for Plants

This invention relates to a granular inoculant composition for plants.

Various microorganisms are known to have beneficial effects on plants, e.g. bacteria of the genera Rhizobium, Pseudomonas, Serratia, Bacillus, Pasteuria, Azotobacter, Azospirillum, Cyanobacteria (blue-green algae) and Arthrobacter, fungi of the genera Gliocladium, Trichoderma, Coniotherium, Verticillium, Paecilomyces, Metarhizium and mycorrhizal fungi. Such microorganisms are introduced into the soil at sowing by the use of inoculant compositions. Although the following description is concerned with Rhizobium granular inoculant compositions, it will be appreciated that similar principles apply to the use of other microorganisms. The term "plants" includes all agricultural crop plants, horticultural plants, trees and bushes.

There is a great potential to increase soil and plant nitrogen supplies through biological $N_2$ fixation (BNF). The ability to fix $N_2$ resides with some free-living microorganisms as well as with others in symbiotic associations. Perhaps the best known relationship is between the bacterium, Rhizobium, and the leguminous plant.

In order to maximise BNF in agriculture, it is necessary to assure the participation of rhizobia with high $N_2$-fixing capabilities in the symbiotic association. This can be accomplished by rhizobia inoculation of leguminous seed. The prime objective of legume seed inoculation is the nodulation of the host plant, and it consists of the introduction of a strain of Rhizobium into the soil at sowing in a form that enables it to:

(a) Remain viable until the host seeding can be infected.

(b) Compete with any indigenous or naturalised rhizobia for infection sites and so form sufficient root nodules to permit maximum $N_2$-fixation in the host legume.

(c) Adequately nodulate its host(s) rapidly over a wide range of environmental conditions.

(d) Persist in soil in numbers sufficient to maintain nodulation of perennial legumes or to achieve prompt nodulation of regenerating annual species.

Peat-based inoculants presently constitute the vast majority of cultures marketed today, and their development is primarily due to their convenience in holding and distributing the rhizobia. Granular inoculants were developed as a direct implant form for sowing into the row beside or beneath the seed and are of particular value under the following circumstances:

a) Conditions where seed inoculation is hazardous because of fragility of the seed coat.

b) Conditions where high soil temperatures exist and granular inoculants may be placed beneath the seed where soil temperatures are lower.

c) Certain epigeal species, particularly soybean and subterranean clover, frequently lift the seed coat out of the soil during emergence of the cotyledons. In such cases, rhizobia may not be deposited in the soil in sufficient numbers if seed inoculation techniques were used.

d) Circumstances when seeds are treated with insecticides or fungicides that endanger rhizobial survival.

e) With small seeded leguminous plants inoculation may not provide sufficient rhizobia to effectively nodulate the plant.

f) Circumstances when high levels of inoculant need to be applied to allow the inoculum strain to complete with naturally occurring rhizobia.

Natural peat granules may be obtained by screening (20-50 mesh) and have previously been used as carriers for rhizobia. However, laboratory studies have shown that such granules only form between 5-10% by weight of dried peat and are therefore an uneconomical source of granular carrier. Likewise, natural clay granules and calcium sulphate granules have also been used for rhizobial inoculants. References to the use of natural peat, natural clay and calcium sulphate granules are as follows:

Reinoculation of lucerne seed. M.E. Fraser. Journal of Applied Bacteriology (1966) 29 (3) 587-594.

Current use of Legume Inoculant Technology. P.M. Williams in Biological Nitrogen Fixation - Ecology, Technology and Physiology, p.173-200. ed. M. Alexander. Plenum Press. New York. 1984.

Surprisingly, we have found that when peat or peat/clay mixtures are mixed with calcium sulphate and then moistened, natural aggregation occurs and on drying, free flowing granules are formed, with very few fine particles remaining.

The present invention thus provides a method of preparing a granular inoculant composition for plants, which comprises mixing at least one species of microorganism capable of having a beneficial effect on the plants with a peat or peat/clay carrier medium; mixing the product with calcium sulphate and moistening to cause aggregation; and drying to produce free-flowing granules.

The calcium sulphate used is preferably $CaSO_4.\frac{1}{2}H_2O$ and the proportion of peat or peat/clay mixture to calcium sulphate is preferably from 50/50 to 75/25 (W/W).

This discovery now allows rhizobial inoculants based on powdered peat to be granulated by mixing with $CaSO_4.\frac{1}{2}H_2O$ followed by the addition of water. If desired, clays may be added prior to moistening the mixture.

Such granular incoulants support a high number of viable Rhizobium species which is maintained during long-term storage (Table 1).

Table 1. Survival of <u>B. japonicum</u> in granular inoculants during storage at room temperature.

| Granule composition | Months Storage | | | | | | | |
|---|---|---|---|---|---|---|---|---|
| | 0 | 1 | 2 | 3 | 4 | 5 | 6 | 7 |
| Peat inoculant/ $CaSO_4 \cdot \frac{1}{2}H_2O$ (1:1 w/w) | $6.5 \times 10^9$ | $6.0 \times 10^9$ | $5.0 \times 10^9$ | $3.8 \times 10^9$ | $2.5 \times 10^9$ | $1.0 \times 10^9$ | $9.0 \times 10^8$ | $7.6 \times 10^8$ |
| Peat inoculant/ $CaSO_4$/calcium montmorillonite | | | | | | | | |
| 1:4:3 w/w | $1.5 \times 10^9$ | $8.0 \times 10^8$ | $5.8 \times 10^8$ | $4.0 \times 10$ | $4.5 \times 10^8$ | $4.0 \times 10^8$ | $3.6 \times 10^8$ | $2.8 \times 10^8$ |
| 1:3:2 w/w | $4.0 \times 10^9$ | $2.8 \times 10^9$ | $2.0 \times 10^9$ | $1.0 \times 10^9$ | $9.0 \times 10^8$ | $8.0 \times 10^8$ | $7.2 \times 10^8$ | $6.5 \times 10^8$ |
| 1:2:1 w/w | $3.8 \times 10^9$ | $2.6 \times 10^9$ | $1.0 \times 10^9$ | $9.5 \times 10^9$ | $8.2 \times 10^8$ | $7.2 \times 10^8$ | $6.8 \times 10^8$ | $5.3 \times 10^8$ |

0 286 351

The invention is illustrated by the following Example.

EXAMPLE

A. Preparation of peat-based inoculants

Selected sedge peat (Fisons) was adjusted to pH 6.5 using calcium hydroxide and calcium carbonate. This was oven dried at 60°C and milled in a hammer mill to pass through a 0.4 mm sieve. 150 g aliquots of powdered peat were sealed into 300 gauge polytene bags and sterilised by gamma radiation (50 KGy). The packs were injected with 120 ml of a pure culture of Rhizobium japonicum, the injection hole re-sealed and the contents intimately mixed prior to incubation for 7 days at 26°C. Such inoculants contain on average $5 \times 10^9$ viable cells $g^{-1}$.

B. Preparation of granular inoculants

1.5 Kg of peat-based inoculant plus 1.2 kg of $CaSO_4$ $\frac{1}{2}H_2O$ (surgical grade, Superfine casting or Fine casting plaster-British Gypsum) were placed in the bowl of a Hobart industrial food mixer and mixed for one minute prior to the addition of 630 ml of tap water which was added at a rate of 800 ml $min^{-1}$ using a peristaltic pump. Mixing continued for 30 seconds and the mixture was then rubbed through a 5 mm sieve before being allowed to harden in air for 12 minutes at 25°C. The grains were then passed through a 2 mm sieve to remove coarse material and 5 kg lots sealed in 500 gauge polythene bags. The bags were incubated for seven days at 26°C. Such granules contained $> 10^9$ cells $g^{-1}$. Granule size distribution and hardening time can be altered by varying the amount of added water (Table 2) - the lower volumes producing more fines and requiring longer hardening times than the higher volumes.

Clays such as Fuller's earth (Laporte Earths), Surrey powder or calcium montmorillonite may be incorporated into the mixture without detrimental effects on rhizobial numbers or aggregation characteristics. In such cases the amount of $CaSO_4$ and volume of $H_2O$ added water are increased accordingly.

## Table 2

| Volume of added $H_2O$ (ml) | Hardening time (min) | Granule size (%) of total | | | |
|---|---|---|---|---|---|
| | | > 2mm | 2 -1 mm | 1-.355 mm | <.355 mm |
| 610 | 15 | 15 | 14 | 48 | 23 |
| 630 | 12 | 18 | 18 | 52 | 12 |
| 650 | 9 | 18 | 22 | 51 | 9 |
| 680 | 6 | 29 | 35 | 31 | 5 |

5

## C. Larger scale preparation of granular inoculants

22kg of peat-based inoculant plus 18kg of $CaSO_4 \cdot \frac{1}{2}H_2O$ were mixed in a ploughshare mixer for one minute prior to the addition of 9.3 litres of water. Mixing was continued until spherical granules were formed. The granules were then passed through a 2mm sieve to remove coarse material and 5kg lots sealed in 500 gauge polythene bags. The bags were incubated for seven days at 26°C. Such granules contained $>5 \times 10^9$ viable cells $9^{-1}$.

## D. Field Trials of Granular Inoculants

The efficacy of granular inoculants prepared as described in Section C was tested under field conditions. Application rates and results are given in Table 3 and 4.

### Table 3
Inoculation of soya in the field at a granule application rate of 10kg $ha^{-1}$.

| Treatment | No. of nodules per plant | Yield ($t.ha^{-1}$) |
|---|---|---|
| Non-inoculated control | 0 | 1.64 |
| Granular inoculant | 121 | 2.95 |

### Table 4
Inoculation of green _Phaseolus_ in the field at a granule application rate of 8kg $ha^{-1}$.

| Treatment | Pod yield ($t.ha^{-1}$) | | | | |
|---|---|---|---|---|---|
| | Nitrogen application rate ($kg.ha^{-1}$) | | | | |
| | 0 | 20 | 40 | 80 | 100 |
| Non-inoculated control | 4.90 | 5.28 | 5.52 | 5.85 | 6.10 |
| Granular inoculant | 5.13 | 5.98 | 6.18 | 6.31 | 6.31 |

## Claims

1. A method of preparing a granular inoculant composition for plants, which comprises mixing at least one species of microorganism capable of having a beneficial effect on the plants with a peat or peat/clay carrier medium; mixing the product with calcium sulphate and moistening to cause aggregation; and drying to produce free-flowing granules.

2. A method according to claim 1, wherein the plants are legume crops and the microorganism is Rhizobium bacteria.

3. A method according to claim 1, wherein the calcium sulphate is in the form of $CaSO_4 \cdot \frac{1}{2} H_2O$.

6

4. A method according to claim 1, wherein the clay is in the form of Fullers Earth, Surrey powder or calcium montmorillonite.

5. A method according to claim 1, wherein the calcium sulphate is used in a proportion of from 25 to 50% by weight, based on the total weight before moistening.

6. A granular inoculant composition prepared by a method according to claim 1.

7. A method of sowing seeds, in which the seeds are sown together with a granular inoculant composition according to claim 6.